# EUROPEAN PATENT SPECIFICATION

(11) **EP 2 468 243 B1**
(45) Date of publication and mention of the grant of the patent: **13.12.2017**
(21) Application number: 10809747.8
(22) Date of filing: 20.08.2010
(51) Int. Cl.: A61K 8/22, A61K 8/02, A61K 8/33, A61K 8/39, A61Q 5/10

(54) **HAIR DYEING METHOD**
HAARFÄRBEZUSAMMENSETZUNG
PROCÉDÉ DE COLORATION CAPILLAIRE

(30) Priority: 21.08.2009 JP 2009191775
(43) Date of publication of application: 27.06.2012
(73) Proprietor: Kao Corporation, Chuo-ku Tokyo 103-8210 (JP)
(72) Inventor: IIJIMA, Makoto, Tokyo 131-8501 (JP); MIYABE, Hajime, Tokyo 131-8501 (JP)
(74) Representative: Hoffmann Eitle
(86) International application number: PCT/JP2010/005151
(87) International publication number: WO 2011/021401

(56) References cited:
- EP-A1- 2 030 606
- WO-A1-2009/054147
- JP-A- 2001 002 537
- JP-A- 2002 284 655
- JP-A- 2003 095 896
- JP-A- 2003 201 225
- JP-A- 2007 308 427
- JP-A- 2009 120 602
- JP-A- 2009 120 604
- JP-A- 2009 161 492

## Description

### FIELD OF THE INVNETION

The present invention relates to a hair dyeing method for dyeing hair using a two-agent type aerosol foam hair dye.

### BACKGROUND OF THE INVENTION

Conventionally, two-agent type aerosol foam hair dyes which discharge hair dyes in the form of foam using propellants have been known (for example, Patent Document 1 and Patent Document 2). As compared with liquid and cream formulations which are generally used formulations for hair dyes, a two-agent type aerosol foam hair dye is advantageous in that the operation is convenient as the hair dye can be discharged only by pressing a button on the aerosol container, in that deterioration of the oxidation dye is prevented because the dye does not contact with outside air, and in that the hair dye can be used dividedly.

However, total application amount of the a two-agent type aerosol foam hair dye at the time of hair dyeing is likely to be smaller compared with the case of conventional hair dyes, due to the apparent large volume of the discharged foam. If the application amount is small, sufficient dyeing is not possible. Besides, it has been known as technical common knowledge that when a two-agent type aerosol foam hair dye is applied on the hair, it is recommended to break the foam by, for example, combing-through (for example, Patent Document 1 and Patent Document 2). Furthermore, it has been known as technical common knowledge that, from the viewpoint of dyeing sufficiently or from the viewpoint of preventing liquid dripping, the hair dye that has been applied and defoamed is prepared as creamy agent, and then is left to stand still, to thereby dye the hair. In addition, since the foam of an aerosol hair dye is formed as a result of the vaporization of a propellant included in the preparation discharged from an aerosol container under pressure, if the foam is once broken, it is impossible to refoam using the propellant.

### PRIOR ART DOCUMENTS

### PATENT DOCUMENT

Patent Document 1: JP-A-H09-136818
Patent Document 2: JP-A-2003-73240

### SUMMARY OF INVENTION

An object of the present invention is to provide a method for dyeing hair, including the steps of discharging a first agent and a second agent from respective containers of an aerosol foam hair dye composed of the first agent and the second agent as described below, applying the discharged agents on hair, and then refoaming the agents on the hair:
- First agent contained in a first aerosol container, including a first agent liquid concentrate containing components (A), (C) and (D), and a propellant:
   (A) an alkali agent,
   (C) a polyoxyethylene alkyl ether type or polyoxyethylene alkenyl ether type nonionic surfactant, having an HLB value of 11 or more, and
   (D) water; and
- Second agent contained in a second aerosol container, including a second agent liquid concentrate containing components (B), (C) and (D), and a propellant:
   (B) hydrogen peroxide,
   (C) a polyoxyethylene alkyl ether type or polyoxyethylene alkenyl ether type nonionic surfactant, having an HLB value of 11 or more, and
   (D) water;
wherein the first agent and/or the second agent further contains an amphoteric surfactant; and wherein the content of surfactant in the liquid concentrates of the first agent and the second agent is 4 to 30% by mass.

### DETAILED DESCRIPTION OF THE INVENTION

A hair dye that has become creamy after foam breaking is not easily spreadable on the hair, and it is difficult to uniformly dye from the root to the top of the hair using the creamy hair dye. Furthermore, it is practically difficult to uniformly and efficiently mix the foam of a first agent and the foam of a second agent that are discharged from different aerosol containers in a short time. On the other hand, in the case of dyeing with an agent that has become creamy after foam breaking, the agent is not easily spreadable, and it is apparently difficult to recognize which part of the hair has or has not been applied with the agent. For these reasons, it has been difficult even for a skilled person to dye uniformly.

The present invention relates to a hair dyeing method using a two-agent type aerosol foam hair dye, by which the hair can be dyed sufficiently, evenly and uniformly, from the root to the top of the hair and over the entire area of application, without difficulties even when an unskillful person uses the method.

The inventors of the present invention have studied, in relation to hair dyeing using a two-agent type aerosol foam hair dye, a method for dyeing hair by refoaming the applied hair dye on the hair, unlike those hair dyeing methods of breaking the foam of the hair dye at the time of application to hair, and then leaving the hair dye to stand still. Surprisingly, the inventors found that the hair dye spreads from the root to the top of hair, and that the hair can be dyed evenly and uniformly, and further sufficiently from the root to the hair end and over the entire area of application, without difficulties even when an unskillful person uses the method.

### <<Definitions>>

The term "refoaming" as used in the present specification means a step of increasing the volume of foam. The term "foam" as used herein does not mean the individual bubbles, but means a collection of bubbles (foam).

The term "hair" as used in the present specification means a concept which includes all of the hair, e.g. head hair, eyebrows, axillary hair, hairpieces and tresses, the term preferably refers to head hair. The term "head hair" as used in the present specification means a concept which refers to the hair that has grown from the scalp, but does not include the hair that has been separated from the scalp, such as hairpieces or tresses. Furthermore, the head hair may be of a doll or of any animal, human hair is preferable.

The term "hair dye" as used in the present specification means a concept which includes, for convenience, not only a hair dye containing a dye, but also a bleaching agent which does not contain a dye.

The term "aerosol foam hair dye" as used in the present specification means an agent which becomes a foamy hair dye when propellant contained in the agent and discharged from an aerosol container under pressure is vaporized. According to the present invention, the timing of formation of foam may be immediately after discharge, or the timing may be slightly delayed after discharge (the form is in creamy or jelly form immediately after discharge, which changes into foam when heat, force or the like is added externally). A formulation which forms a foam immediately after discharge is more preferable.

According to the present specification, an agent which becomes foam immediately after discharge, an agent which becomes creamy or jelly form immediately after discharge, and an agent which has changed as foam from creamy or jelly form, may be collectively referred to as an "agent".

The term "hair dyeing" as used in the present specification means a concept which includes, for convenience, not only the step of hair dyeing using a hair dying agent, but also includes the step of bleaching using a bleaching agent.

The term "liquid concentrate" shall refer to whole components other than propellant in the aerosol container.

The first agent and the second agent of the subject two-agent type aerosol foam hair dye are necessary to be filled separately in respective aerosol containers together with a propellant, because the first agent and the second agent are necessary to be mixed immediately before use. Therefore, when the first agent and the second agent are discharged from separate aerosol containers by means of pressure generated from vaporization of the propellant, a foam of the first agent and a foam of the second agent are separately formed. The separately formed foams are mixed before being applied to the hair, or mixed on the hair after being applied. At the time of this mixing, while the vaporized propellant is scattered and lost, the remaining liquid concentrate of the first agent and the second agent, which have been restored to liquid concentrate, or which are still foam, are mixed. According to such embodiments of use, the term "mixed liquid" itself means in the present specification, for convenience, a liquid obtained from mixing the liquid concentrates of the first agent and the second agent (provided that the liquid concentrates of the first agent and the second agent contained in separate aerosol foamer containers are not mixed within the containers). Similarly, the foam obtained by mixing the foam of the first agent and the second agent will also be referred to, for convenience, simply as "foam of the mixed liquid" in the present specification.

### • Procedure for hair dyeing

According to the hair dyeing method of the present invention, the hair is preferable to be combed in advance before the application of the discharged agent. Thereby, the hair is not easily entangled during the step of refoaming the agent, in order to prevent the foam hair dye being scatter away. Furthermore, blocking step, which is normally performed during application of a hair dye composition, is not necessary to be performed, preferably the blocking operation is not performed. Thereby, the step of applying the foamy hair dye described below to the hair, or the step of refoaming the hair dye can be facilitated.

Preferably no hairdressing agent is applied to the hair to which the foam hair dye will be applied in the step of hair dyeing treatment, from the viewpoint of obtaining an even and sufficient hair dyeing effect and also preventing liquid dripping. Furthermore, from the viewpoint of obtaining an even and sufficient hair dyeing effect without causing dilution of the mixed liquid, and also preventing liquid dripping, it is preferable to apply the hair dye to dry hair. In the case of washing hair immediately before the hair dyeing treatment, it is preferable to dry the hair before the hair dyeing treatment is carried out. Drying the hair shall mean eliminating the liquid which contains mainly water that has been applied during washing hair to the extent that the liquid at least does not drip in the natural state. Specifically, it is preferable to towel-dry the hair, or to dry the hair using hair dryer.

From the viewpoint of sufficiently mixing the liquid concentrate and the propellant in an aerosol container, and preparing foams of the first agent and the second agent discharged separately from respective aerosol containers which can be easily applied to the hair, it is preferable to shake the aerosol containers before discharging the first agent and the second agent from the respective aerosol containers.

With regard to the degree of shaking, the rate of shaking is preferably 0.5 to 5 reciprocations, more preferably 1 to 4 reciprocations, and even more preferably 2 to 3 reciprocations, per second. The amplitude of shaking is preferably 5 to 50 cm, more preferably 10 to 40 cm, and even more preferably 20 to 30 cm. The number of shaking is preferably 2 to 20 reciprocations, more preferably 3 to 15 reciprocations, and even more preferably 4 to 10 reciprocations. Thereafter, it is preferable to discharge the hair dye without long time interval (preferably within 10 minutes, more preferably within 5 minutes, and even more preferably within 3 minutes).

The two-agent type aerosol foam hair dye is used in a manner that the first agent and the second agent are mixed immediately before use, and the hair dyeing treatment is carried out. The mixing of the first agent and the second agent in the present invention is carried out by any of the following methods:
1) the first agent and the second agent are separately discharged from the aerosol containers, and then are mixed before being applied to the hair;
2) the first agent and the second agent are separately discharged from the aerosol containers, and then are mixed at the time of being applied to the hair; and
3) the first agent and the second agent are separately discharged from the aerosol containers, and then are applied to the hair, followed by mixing on the hair.
A preferred mixing ratio of the first agent and the second agent is approximately 2:1 to 1:2, and more preferably almost 1:1, based on amount of the liquid concentrates.

The first agent and the second agent thus discharged are at first taken on the hand or on a brush and then applied to the hair, or the discharged agents are directly applied to the hair. In the case of using hand, it is preferable for the user to wear gloves. Since the blocking operation, which is generally performed for application of hair dye compositions, is unnecessary in the hair dyeing method of the present invention, the hair dye can be applied in a short time. Therefore, the hair dye may be applied from anywhere of the hair, and it is unnecessary to apply the hair dye from the nape of the neck as performed in the case of the conventional liquid or cream type two-agent type hair dye compositions. The hair dye may be applied starting from areas of concern, and it is preferable to apply the hair starting from the hairline or the parting area of hair.

It is preferable to discharge the foam, the size of which is close to lemon-size, because it is handy size and easy to hold the foam in one hand, and it is easy to apply the foam to the hair with the hand. In this case, the aerosol container is operated with one hand, and the foam is held in the other hand. The foam once held in the hand is applied to the hair, subsequently the foam is discharged on the hand again, and applying the foam to the hair, and this series of operations are repeated. The series of operations can be carried out very conveniently in a short time.

Furthermore, the area to which the foam is applied may be the entire area of the hair, or may be some specific parts.

Subsequently, the applied foam is refoamed on the hair. Gas may be injected, or an instrument such as a vibrator or a brush may be used, or fingers may be used for refoaming. However, it is more preferable to use fingers because the foam can be sufficiently spread to the root of the hair by using fingers. The rate of foaming using a vibrator or a brush or using fingers is preferably controlled so that the foam does not scatter away.

Here, the timing of refoaming may be after the foam has completely disappeared, may be in the middle of the disappearance of the foam, or may be before the applied foam changes. Alternatively, the timing of refoaming may be after the foam has been completely applied over the entire targeted area for applying the foam, or may be during the application. The step of refoaming may be carried out continuously once, or may be repeated several times intermittently. In the subject invention, if the vibrator or brush or the fingers used for refoaming are continuously in contact with some of the hair, or if the vibrator or brush or the fingers are only momentarily separated (i.e. they are brought again into contact within one second), this step of refoaming shall be considered as "continuous". Accordingly, the targeted site for applying hair dye may be observed, and the hair dye may be refoamed appropriately. When the foam that is disappearing is refoamed, the hair dye can be spread out conveniently irrespective of the nature of the foam. Thus, even an unskillful person can securely dye the targeted area for applying the hair dye. In addition, the user can uniformly mix the foam of the first agent and the second agent unconsciously by the step of refoaming. Furthermore, by refoaming the agents early, and by rapidly spreading the agents over the targeted area for applying the agents, difference of exposure time with the agents in an area of application can be prevented, and thus unevenness can be prevented. The timing is preferably within 5 minutes, more preferably within 3 minutes, and even more preferably within one minute, after completion of the application of the discharged agents to the hair.

Hereinafter, a specific example of a preferable procedure for the steps including the discharge of agents, application to the hair, and foaming again, is described with respect to partial dyeing and whole head dyeing.

### • Partial dyeing

### Procedure example 1)

The agents are discharged in appropriate amounts on one hand or on a brush, and are applied on some parts of the hair. The step of refoaming the agents is carried out once for one second to 10 minutes, and preferably for 3 seconds to 3 minutes.

### Procedure example 2)

The agents are discharged in appropriate amounts on one hand or on a brush, and are applied on some parts of the hair. The step of refoaming the agents is carried out 2 to 30 times, for one second to 10 minutes, and preferably for 3 seconds to 3 minutes, per one step. The step is carried out for 2 seconds to 20 minutes, and preferably for 5 seconds to 5 minutes, in total.

### • Whole head dyeing

### Procedure example 3)

The agents are discharged in appropriate amounts on one hand, and are applied on some parts of the hair. The step of refoaming the agents is carried out once for 3 seconds to 10 minutes, and preferably for 5 seconds to 3 minutes. This step is repeated to apply the agents over the whole head.

### Procedure example 4)

The agents are discharged in appropriate amounts on one hand, and are applied on some parts of the hair. The step of refoaming the agents is carried out once for 3 seconds to 10 minutes, and preferably for 5 seconds to 3 minutes. This step is repeated to apply the agents over the whole head, and then the step of refoaming the agents is carried out once for 3 seconds to 10 minutes, and preferably for 5 seconds to 3 minutes. Furthermore, the agents are discharged in appropriate amounts on one hand, and are additionally applied on some parts of the hair. The operation of refoaming the agents is carried out once over the whole head for 3 seconds to 10 minutes, and preferably for 5 seconds to 3 minutes.

### Procedure example 5)

The agents are discharged in appropriate amounts on one hand, and are applied on some parts of the hair. The step of refoaming the agents is carried out once for 3 seconds to 10 minutes, and preferably for 5 seconds to 3 minutes. This step is repeated to apply the agents over the whole head. After completion of the application over the whole head, the operation of refoaming the agents is carried out once for 3 seconds to 10 minutes, and preferably for 5 seconds to 5 minutes.

### Procedure example 6)

The agents are discharged in appropriate amounts on one hand, and are applied on some parts of the hair. The operation of refoaming the agents is carried out once for 3 seconds to 10 minutes, and preferably for 5 seconds to 3 minutes. This step is repeated to apply the agents over the whole head. After completion of the application over the whole head, the step of refoaming the agents over the whole head is carried out 2 to 30 times, for 3 seconds to 10 minutes, and preferably for 5 seconds to 3 minutes, per one step. The entire operation is carried out for 6 seconds to 20 minutes, and preferably for 10 seconds to 5 minutes, in total.

### Procedure example 7)

The agents are discharged in appropriate amounts on a brush, and are applied on some parts of the hair. This step is repeated to apply the agents over the whole head, and the step of refoaming the agents is carried out over the whole head using the same brush for 3 seconds to 10 minutes, and preferably for 5 seconds to 5 minutes.

### Procedure example 8)

The agents are discharged in appropriate amounts on a brush, and are applied on some parts of the hair. The step of refoaming the agents is carried out once using the same brush or with the hands for 3 seconds to 10 minutes, and preferably for 5 seconds to 3 minutes. This step is repeated to apply the agents over the whole head. After completion of the application over the whole head, the step of refoaming the agents is carried out once using the same brush or with the hands for 3 seconds to 10 minutes, and preferably for 5 seconds to 5 minutes.

The area in which the agents are refoamed may be the entire hair, or may be some specific parts only. When the agents are refoamed over the entire hair, even if the user forgets to apply the foam on the parts where it is difficult to confirm, such as the hair in the back of the head, the foam can be spread through, and generation of undyed parts can be prevented. When the agents are refoamed only in specific parts by partial dyeing, the boundaries of dyeing can be gradated, and thus a natural finish is obtained. Furthermore, when the step of refoaming is carried out again, the area where the foam has been spread through can be very easily recognized, and thereby the generation of undyed parts, which is intended for dyeing, can be prevented.

The foam is washed away after a time gap of about 3 to 60 minutes, and preferably about 5 to 45 minutes from the completion of the application of the foam. According to the present invention, the time after the completion of the application of foam means the total required time from the completion of the application of all the foam over the whole head or in desired parts to the washing away of the foam. Thus, this concept includes the time required for refoaming in addition to the step of leaving the agents to stand still. Thereafter, the hair is subjected to appropriate shampooing or conditioning, and then the hair is washed with water and dried.

### <<Aerosol foam hair dye>>

The aerosol foam hair dye used in the present invention includes a first agent liquid concentrate which contains (A) an alkali agent, (C) a polyoxyethylene alkyl ether type or polyoxyethylene alkenyl ether type nonionic surfactant, having an HLB value of 11 or more, and (D) water, and a second agent liquid concentrate which contains (B) hydrogen peroxide, (C) a polyoxyethylene alkyl ether type or polyoxyethylene alkenyl ether type nonionic surfactant, having an HLB value of 11 or more, and (D) water;
wherein the first agent and/or the second agent further contains an amphoteric surfactant; and wherein the content of surfactant in the liquid concentrates of the first agent and the second agent is 4 to 30% by mass.

### [(A) Alkali agent]

For example, ammonia, an alkanolamine such as ethanolamine, sodium hydroxide, and potassium hydroxide can be used as the alkali agent contained in the first agent. Furthermore, ammonium salts such as ammonium carbonate, ammonium hydrogen carbonate, and ammonium chloride; carbonic acid salts such as potassium carbonate, sodium carbonate, potassium hydrogen carbonate, and sodium hydrogen carbonate; and the like can be appropriately added as buffer agents.

The pH of the mixed liquid of the liquid concentrate of the first agent and the second agent according to the aerosol foam hair dye used in the present invention is preferably 8 to 11, and more preferably 9 to 11. The content of the alkali agent in the mixed liquid is appropriately selected for adjusting the pH of the mixed liquid to the above pH value, and the content is, although the content may vary depending on the type of the alkali agent, preferably 0.1% to 10% by mass.

### [(B) Hydrogen peroxide]

The content of hydrogen peroxide in the second agent liquid concentrate is preferably 1% to 9% by mass, and more preferably 3% to 6% by mass, and the content of hydrogen peroxide in the mixed liquid of the first agent liquid concentrate and the second agent liquid concentrate is preferably 1% to 6% by mass, and more preferably 2% to 5% by mass. Furthermore, the pH of the second agent liquid concentrate is preferably adjusted to 2 to 6, and more preferably set to pH 2.5 to 4, in order to prevent decomposition of hydrogen peroxide.

### [(C) Surfactant]

The polyoxyethylene alkyl ether type- or polyoxyethylene alkenyl ether type nonionic surfactants having an HLB value of 11 or more preferably have an alkyl group or alkenyl group having 10 to 20 carbon atoms, and more preferably 12 to 18 carbon atoms. Furthermore, this alkyl group or alkenyl group is preferably straight-chained. It is also preferable that the polyoxyethylene group have an average addition mole number of 1 to 40, and more preferably 4 to 30.

The polyoxyethylene alkyl ether type nonionic surfactants are preferred from the viewpoint of providing a preferable balance between the foamability by means of a propellant, the ease of refoaming, and the stability in the presence of a propellant.

### Amphoteric surfactant

Examples of the amphoteric surfactant include carbobetaines, amidobetaines, sulfobetaines, hydroxysulfobetaines, amidosulfobetaines, phosphobetaines and imidazoliniums having an alkyl group, alkenyl group or acyl group having 8 to 24 carbon atoms, and among them, carbobetaines and sulfobetaines are preferred. Preferred examples of the amphoteric surfactant include lauramidopropylbetaine, coconut oil fatty acid amidopropylbetaine, lauryldimethylaminoacetic acid betaine, and laurylhydroxysulfobetaine.

The content of the surfactant in the liquid concentrates of the first agent and the second agent is 4 to 30% by mass, preferably 5% to 15%, even more preferably 4% to 10% by mass, and even more preferably 5% to 8% by mass, from the viewpoint of making foams of the first agent and the second agent discharged from respective aerosol containers which can be easily applied to the hair, easily mixed after foaming, and easily refoam after being applied to the hair.

Any type of the surfactant exemplified below can be used, from the viewpoint of making foams of the first agent and the second agent discharged from respective aerosol containers which can be easily applied to the hair, easily mixed after foaming, and easily refoam after being applied to the hair.

### • Anionic surfactant

Examples of the anionic surfactant include sulfuric acid ester salts such as alkyl sulfates and polyoxyalkylene alkyl ether sulfates; carboxylic acid salts such as fatty acid salts, N-acylamino acid salts (N-acyl sarcosinate, N-acyl glutamate, N-acyl glycinate, and the like), salts of alkyl succinates or alkenyl succinates, polyoxyalkylene alkyl ether carboxylates, and fatty acid amide ether acetates; phosphoric acid ester salts such as alkyl phosphates and alkyl ether phosphates; and sulfonic acid salts such as polyoxyalkylene alkyl ether sulfosuccinates, isethionic acid fatty acid ester salts, acyl taurates, alkyl benzenesulfonates, α-olefin sulfonates, and alkane sulfonates. Among these, alkyl sulfates and polyoxyalkylene alkyl sulfates are preferred, and preferred are salts thereof having an alkyl group having 10 to 24 carbon atoms, even more preferably 12 to 18 carbon atoms. Preferably, this alkyl group is straight-chained. Furthermore, polyoxyalkylene alkyl sulfates, inter alia, polyoxyethylene alkyl sulfates are more preferred, and among them, more preferred are salts thereof in which the average addition mole number of the oxyethylene group is 1 to 10, and even more preferably 2 to 5. Furthermore, N-acyl amino acid salts and ether carboxylic acid salts are also preferable, and among them, N-acyl glutamates having an acyl group having 10 to 18 carbon atoms, and polyoxyethylene alkyl carboxylates in which the alkyl group has 10 to 18 carbon atoms and the average addition mole number of the oxyethylene group is 3 to 15, are preferred.

### • Nonionic surfactant

Examples of the nonionic surfactant include polyoxyalkylene addition type-, sugar addition type-, glycerin addition type-, and alkanolamide addition type nonionic surfactants. Examples of the polyoxyalkylene addition type nonionic surfactant include ethers such as polyoxyethylene alkyl ethers and polyoxyethylene alkenyl ethers; and esters such as polyoxyethylene fatty acid esters. Examples of the sugar addition type nonionic surfactant include sugar ethers such as alkyl polyglucosides; sugar esters such as sucrose fatty acid esters, and sorbitan fatty acid esters; and fatty acid glycolamides. Examples of the glycerin addition type nonionic surfactant include alkyl glyceryl ethers, and glycerin fatty acid esters. Examples of the alkanolamide type nonionic surfactant include fatty acid monoalkanolamides, and fatty acid diethanolamides.

Here, the polyoxyethylene alkyl ether type- or polyoxyethylene alkenyl ether type nonionic surfactants preferably have an alkyl group or alkenyl group having 10 to 20 carbon atoms, and more preferably 12 to 18 carbon atoms. Furthermore, this alkyl group or alkenyl group is preferably straight-chained. It is also preferable that the polyoxyethylene group have an average addition mole number of 1 to 40, and more preferably 4 to 30. The polyoxyethylene fatty acid ester type nonionic surfactants preferably have a fatty acid residue having 10 to 20 carbon atoms, and more preferably 12 to 18 carbon atoms, and the fatty acid residue is preferably saturated and/or straight-chained. The alkyl polyglucoside type nonionic surfactants preferably have an alkyl group having 8 to 18 carbon atoms, more preferably 8 to 14 carbon atoms, and even more preferably 9 to 11 carbon atoms, and this alkyl group is preferably straight-chained. The average degree of polymerization of the glucoside is preferably 1 to 5, and more preferably 1 to 2. Examples of the sucrose fatty acid ester type nonionic surfactants include glycol monofatty acid esters and glycol difatty acid esters, and it is preferable that the fatty acid residue have 12 to 22 carbon atoms. The alkyl glyceryl ether type nonionic surfactants preferably have an alkyl group having 8 to 22 carbon atoms, and more preferably 12 to 18 carbon atoms. Furthermore, the average addition mole number of glycerin is preferably 1 to 4, and more preferably 1 to 2. The glycerin fatty acid ester type nonionic surfactants preferably have a fatty acid residue having 10 to 20 carbon atoms, and more preferably 12 to 18 carbon atoms, and the alkyl group is preferably straight-chained. Also, the average addition mole number of glycerin is preferably 1 to 10, and more preferably 1 to 4. The fatty acid monoalkanolamide or fatty acid diethanolamide type nonionic surfactants preferably have a fatty acid residue having 12 to 22 carbon atoms.

### • Cationic surfactant

A compound represented by the following formula (1) can be used as the cationic surfactant. 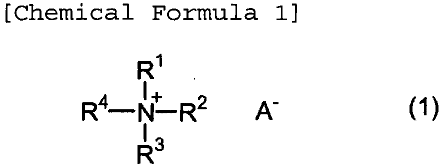 [wherein R¹, R², R³ and R⁴ each independently represent a hydrocarbon group which may be substituted with a substituent; at least one of R¹ and R² has 8 to 36 carbon atoms, while the other has 1 to 7 carbon atoms, or R³ and R⁴, together with the adjacent nitrogen atom, may be joined together to form a 5-membered to 7-membered ring which may be substituted with an alkyl group having 1 to 4 carbon atoms, and may contain a nitrogen atom, an oxygen atom or a sulfur atom as a heteroatom in addition to the nitrogen atom; and A⁻ represents an anion.]

Examples of the hydrocarbon group include a straight-chained or branched alkyl group, a straight-chained or branched alkenyl group, an aryl group, and an aralkyl group, and examples of the substituent include a hydroxyl group, an alkoxy group, an aryloxy group, an epoxy group, an amino group, a mono- or dialkylamino group, a trialkylammonium group, a fatty acid amide group, and a fatty acid ester group. Furthermore, examples of the ring that is formed by joining R³ and R⁴ together with the adjacent nitrogen atom include a morpholine ring, an imidazoline ring, a piperazine ring, a piperidine ring, and a pyrrolidine ring.

Examples of the anion include chloride ion, bromide ion, iodide ion, methylsulfate ion, ethylsulfate ion, acetate ion, phosphate ion, sulfate ion, lactate ion, and saccharin ion.

Specific examples of the cationic surfactant include cetyltrimethylammonium chloride, stearyltrimethylammonium chloride, isostearyltrimethylammonium chloride, lauryltrimethylammonium chloride, behenyltrimethylammonium chloride, octadecyltrimethylammonium chloride, cocoyltrimethylammonium chloride, cetyltrimethylammonium bromide, stearyltrimethylammonium bromide, lauryltrimethylammonium bromide, isostearyllauryldimethylammonium chloride, dicetyldimethylammonium chloride, distearyldimethylammonium chloride, dicocoyldimethylammonium chloride, γ-gluconamidopropyldimethylhydroxyethylammonium chloride, di(polyoxyethylene(2))oleylmethylammonium chloride, dodecyldimethylethylammonium chloride, octyldihydroxyethylmethylammonium chloride, tri(polyoxyethylene(5))stearylammonium chloride, polyoxypropylenemethyldiethylammonium chloride, lauryldimethyl(ethylbenzyl)ammonium chloride, behenic acid amidopropyl-N,N-dimethyl-N-(2,3-dihydroxypropyl)ammonium chloride, tallow dimethylammoniopropyltrimethylammonium dichloride, and benzalkonium chloride.

The cationic surfactant is preferably a monoalkyltrimethylammonium salt or a dialkyldimethylammonium salt, wherein R¹ is, or both R¹ and R² are, preferably a straight-chained or branched alkyl group having 8 to 30 carbon atoms, more preferably 10 to 24 carbon atoms, and even more preferably 12 to 18 carbon atoms; and the rest are methyl groups . Among them, a monoalkyltrimethylammonium salt is preferred.

### • Semipolar surfactant

Examples of the semipolar surfactant include alkylamine oxides.

### [(D) Water]

Water is an essential component of the hair dye liquid concentrate. Since the hair to be applied in the hair dyeing method of the present invention is dry hair as described above, water in the hair dye is likely to be absorbed between the time point of applying the hair dye to the hair and the time point of refoaming. Therefore, the content of water may vary depending on the content of the surfactant or oily agents and the mass ratios thereof; from the viewpoint of making appropriate foam at the time of refoaming, the content of water is preferably 70% to 96% by mass, more preferably 80% to 94% by mass, and even more preferably 87% to 92% by mass, in the total amount of the first agent liquid concentrate and the second agent liquid concentrate.

### [Dye]

When the aerosol foam hair dye used in the present invention is a hair bleaching agent, the agent does not contain a dye, and when the aerosol foam hair dye does not contain a dye, the agent contains an oxidation dye intermediate or a direct dye in the first agent.

### (Oxidation dye intermediate)

Publicly-known precursors or publicly-known couplers, which are conventionally used in hair dyes, can be used as an oxidation dye intermediate. Examples of the precursor include paraphenylenediamine, toluene-2,5-diamine, 2-chloroparaphenylenediamine, N-methoxyethylparaphenylenediamine, N,N-bis(2-hydroxyethyl)-paraphenylenediamine, 2-(2-hydroxyethyl)-paraphenylenediamine, 2,6-dimethylparaphenylenediamine, 4,4'-diaminodiphenylamine, 1,3-bis(N-(2-hydroxyethyl)-N-(4-aminophenyl)amino)-2-propan ol, PEG-3,3,2'-paraphenylenediamine, paraaminophenol, paramethylaminophenol, 3-methyl-4-aminophenol, 2-aminomethyl-4-aminophenol, 2-(2-hydroxyethylaminomethyl)-4-aminophenol, ortho-aminophenol, 2-amino-5-methylphenol, 2-amino-6-methylphenol, 2-amino-5-acetamidophenol, 3,4-diaminobenzoic acid, 5-aminosalicylic acid, 2,4,5,6-tetraaminopyrimidine, 2,5,6-triamino-4-hydroxypyrimidine, 4,5-diamino-1-(4'-chlorobenzyl)pyrazole, 4,5-diamino-l-hydroxyethylpyrazole, and salts thereof.

Furthermore, examples of the coupler include metaphenylenediamine, 2,4-diaminophenoxyethanol, 2-amino-4-(2-hydroxyethylamino)anisole, 2,4-diamino-5-methylphenethol, 2,4-diamino-5-(2-hydroxyethoxy)toluene, 2,4-dimethoxy-1,3-diaminobenzene, 2,6-bis(2-hydroxyethylamino)toluene, 2,4-diamino-5-fluorotoluene, 1,3-bis(2,4-diaminophenoxy)propane, meta-aminophenol, 2-methyl-5-aminophenol, 2-methyl-5-(2-hydroxyethylamino)phenol, 2,4-dichloro-3-aminophenol, 2-chloro-3-amino-6-methylphenol, 2-methyl-4-chloro-5-aminophenol, N-cyclopentyl-meta-aminophenol, 2-methyl-4-methoxy-5-(2-hydroxyethylamino)phenol, 2-methyl-4-fluoro-5-aminophenol, resorcin, 2-methylresorcin, 4-chlororesorcin, 1-naphthol, 1,5-dihydroxynaphthalene, 1,7-dihydroxynaphthalene, 2,7-dihydroxynaphthalene, 2-isopropyl-5-methylphenol, 4-hydroxyindole, 5-hydroxyindole, 6-hydroxyindole, 7-hydroxyindole, 6-hydroxybenzomorpholine, 3,4-methylenedioxyphenol, 2-bromo-4,5-methylenedioxyphenol, 3,4-methylenedioxyaniline, 1-(2-hydroxyethyl)amino-3,4-methylenedioxybenzene, 2,6-dihydroxy-3,4-dimethylpyridine, 2,6-dimethoxy-3,5-diaminopyridine, 2,3-diamino-6-methoxypyridine, 2-methylamino-3-amino-6-methoxypyridine, 2-amino-3-hydroxypyridine, 2,6-diaminopyridine, and salts thereof.

Two or more kinds of the precursor and the coupler may be used in combination, or the agents may be used together with a direct dye such as described below. The contents of the precursor and the coupler are respectively preferably 0.01% to 5% by mass, and more preferably 0.1% to 4% by mass, based on the total amount of the first agent liquid concentrate and the second agent liquid concentrate.

### (Direct dye)

Examples of the direct dye include an acidic dye, a nitro dye, a disperse dye, and a basic dye. Examples of the acidic dye include Blue No. 1, Violet No. 401, Black No. 401, Orange No. 205, Red No. 227, Red No. 106, Yellow No. 203, and Acid Orange 3. Examples of the nitro dye include 2-nitro-p-phenylenediamine, 2-amino-6-chloro-4-nitrophenol, 3-nitro-p-hydroxyethylaminophenol, 4-nitro-o-phenylenediamine, 4-amino-3-nitrophenol, 4-hydroxypropylamino-3-nitrophenol, HC Blue No. 2, HC Orange No. 1, HC Red No. 1, HC Yellow No. 2, HC Yellow No. 4, HC Yellow No. 5, HC Red No. 3, and N,N-bis-(2-hydroxyethyl)-2-nitro-p-phenylenediamine. Examples of the disperse dye include Disperse Violet 1, Disperse Blue 1, and Disperse Black 9. Examples of the basic dye include Basic Blue 99, Basic Brown 16, Basic Brown 17, Basic Red 76, Basic Red 51, Basic Yellow 57, Basic Yellow 87, and Basic Orange 31.

Two or more kinds of the direct dye may be used in combination, or the direct dye may be used together with an oxidation dye intermediate. The content of the direct dye is preferably 0.001% to 5% by mass, and more preferably 0.01% to 3% by mass, based on the total amount of the first agent liquid concentrate and the second agent liquid concentrate.

### [Solvent]

From the viewpoint of preferably controlling the volatilization of water between the time point of applying discharged agents to the hair and the time point of refoaming to thereby improve foaming when the discharged agents are refoamed, and from the viewpoint of sufficient penetration of the hair dye into the hair to thereby provide preferable dyeing, it is preferable to incorporate a solvent into the first agent liquid concentrate and the second agent liquid concentrate. It is preferable to incorporate a lower alcohol such as ethanol, n-propanol or isopropanol, or a hydrophilic non-volatile solvent as the solvent. Among these, a hydrophilic non-volatile solvent is preferred. The hydrophilic non-volatile solvent is preferably a compound showing low anti-foaming action, and examples thereof include polyols and lower alkyl ethers of polyols. The polyols are preferably polyols having 2 to 6 carbon atoms, and the examples include glycerin, propylene glycol, dipropylene glycol, 1,3-butanediol, ethylene glycol, diethylene glycol, isoprene glycol, and sorbitol. Examples of the lower alkyl ethers of polyols include the mono-lower alkyl ethers and poly-lower alkyl ethers (for example, di-lower alkyl ethers) of the polyols described above. Among them, preferred examples include monomethyl ether or monoethyl ethers of polyols, and the specific examples include ethylene glycol monomethyl ether, ethylene glycol monoethyl ether, diethylene glycol monomethyl ether, and diethylene glycol monoethyl ether.

These solvents can be used alone or can be used in combination of two or more. The content of the solvent is preferably 0.1% to 15% by mass, more preferably 0.2% to 10% by mass, even more preferably 0.5% to 5% by mass, and even more preferably 1% to 3% by mass, based on the total amount of the first agent liquid concentrate and the second agent liquid concentrate.

### [Oil agent]

It is preferable to further incorporate an oil agent into the first agent liquid concentrate and/or the second agent liquid concentrate, from the viewpoint of stabilizing the discharged foam. Examples of such an oil agent include higher alcohols such as myristyl alcohol, cetyl alcohol, stearyl alcohol, behenyl alcohol, 2-octyldodecanol, and cetostearyl alcohol; fatty acids such as capric acid, lauric acid, myristic acid, palmitic acid, stearic acid, behenic acid, oleic acid, coconut oil fatty acids, isostearic acid, and isopalmitic acid; hydrocarbon oils such as squalene, squalane, liquid paraffin, liquid isoparaffin, and cycloparaffin; and ester oils such as isopropyl palmitate, isopropyl myristate, octyldodecyl myristate, hexyl laurate, cetyl lactate, propylene glycol monostearate, oleyl oleate, hexadecyl 2-ethylhexanoate, isononyl isononanoate, and tridecyl isononanoate. Among these, higher alcohols, more preferably straight-chained higher alcohols are preferred, and the higher alcohols preferably have 12 to 22 carbon atoms, and more preferably have 14 to 18 carbon atoms. Specifically, myristyl alcohol, cetyl alcohol, and stearyl alcohol are preferred.

The content of the oil agent in the total amount of the first agent liquid concentrate and the second agent liquid concentrate may vary depending on the type of the oil agent, the content is preferably 0.01% to 10% by mass, more preferably 0.05% to 5% by mass, even more preferably 0.1% to 3% by mass, even more preferably 0.3% to 1.5% by mass, and even more preferably 0.45% to 1% by mass. When the content is within the above range, the first agent and the second agent discharged from the aerosol containers can be respectively easily applied to the hair, and can be easily mixed with each other, while, preferable foaming can be provided when the agents are applied to the hair and then are refoamed, so that the hair dye can be easily spread and the foam retention after refoaming is enhanced, as a result, satisfactory dyeing can be provided.

Furthermore, from the above described viewpoint, the ratio of the surfactant and the oil agent, contained in the first agent liquid concentrate and the second agent liquid concentrate, as a whole, may vary depending on the type of the surfactant and the type of the oil agent, the mass ratio of (surfactant/oil agent) is preferably 2 to 3000, more preferably 3 to 1000, and even more preferably 5 to 100.

### [Other optional components]

The first agent liquid concentrate and the second agent liquid concentrate can contain, in addition to the components described above, other components that are conventionally used as raw materials for cosmetics. Examples of such optional components include animal and plant fats and oils, natural or synthetic polymers, ethers, protein derivatives, hydrolyzed proteins, amino acids, antiseptics, chelating agents, stabilizers, antioxidants, plant extracts, herbal medicine extracts, vitamins, fragrances, and ultraviolet absorbers.

### [Viscosity]

The viscosity of the first agent liquid concentrate and the second agent liquid concentrate is respectively preferably 1 to 8000 mPa·s, more preferably 5 to 6000 mPa·s, and even more preferably 10 to 4000 mPa·s, from the viewpoint of discharging the first agent and the second agent separately from the aerosol containers, and making the agents compatible with the hair after being applied to the hair so that the hair dye can sufficiently penetrate into the hair to secure satisfactory dyeing, while improving the foamability at the time of refoaming, and from the viewpoint of making easily spreadable foam obtained by refoaming.

Furthermore, the viscosity of the mixed liquid in the case of mixing the total amounts of the first agent liquid concentrate and the second agent liquid concentrate is preferably 1 to 10000 mPa·s, more preferably 5 to 8000 mPa·s, and even more preferably 10 to 5000 mPa·s, from the viewpoint of making the agents compatible with the hair after being applied to the hair so that the hair dye can sufficiently penetrate into the hair to secure satisfactory dyeing, while improving the foamability at the time of refoaming, and from the viewpoint of making easily spreadable foam obtained by refoaming.

The viscosity as used herein refers to the value measured by a Brookfield type viscometer (rotation speed 30 rpm, for 60 seconds, 30°C). The measurement is carried out using at first Rotor No. 1, and when the measured value is larger than the upper limit, Rotor No. 2 is used. If the measured value is still larger than the upper limit, Rotor No. 3 is used. If the measured value is still larger than the upper limit, Rotor No. 4 is used. When the measured value within the respective measurement ranges of the rotors can be obtained, the values shall be defined as the measurement results, and rotors of subsequent numbers are not used. The measurement is carried out in a constant temperature bath set at the measurement temperature. A container in which a sample for measurement is placed in advance is left to stand still in the constant temperature bath, and the temperature of the sample for measurement is adjusted to the measurement temperature. The measurement is carried out using the mixed liquid which is obtained by mixing the first agent and the second agent and preparing a uniform mixed liquid immediately before the measurement. Heat of reaction is generated as a result of mixing, and the temperature may not match up with the measurement temperature. However, the temperature change due to the reaction heat shall not be taken into account.

### [Propellant]

The propellant is an agent for discharging the contained hair dye from the aerosol container in the form of foam, and generally used propellant can be used. Examples thereof include liquefied petroleum gas (LPG), dimethyl ether (DME), carbon dioxide, nitrogen gas, and mixtures thereof. Furthermore, alternative chlorofluorocarbons such as HFC-152a can also be used. Among these, from the viewpoint of lowering the viscosity of the hair dye liquid concentrate to improve the miscibility with gas, and thereby making the step of foaming easier when the hair dye is refoamed, liquefied petroleum gas (LPG), dimethyl ether (DME) or a mixture thereof is preferred, and it is more preferable that the propellant contain dimethyl ether (DME). The proportion of dimethyl ether (DME) in the propellant is preferably 1% to 99% by mass, more preferably 2% to 97% by mass, and even more preferably 5% to 95% by mass.

### [Aerosol container]

A first aerosol container for filling the first agent liquid concentrate and a propellant, and a second aerosol container for filling the second agent liquid concentrate and a propellant may be produced from any material as long as the aerosol containers have a mechanism for discharging a liquid concentrate in the form of foam, can withstand the pressure of the propellant, and are appropriate for storage. Examples of the containers include glass bottles and metal cans. In the case of a metal can, it is preferable to use a double-wall container in which an inner pouch is attached to the inner surface of the can so as to prevent corrosion of metal by the contents.

The internal pressure (20°C) of the aerosol container is preferably 0.01 to 2 MPa, more preferably 0.02 to 1.5 MPa, and even more preferably 0.05 to 1 MPa. From the viewpoint of obtaining a foam that is easily applicable to the hair, uniformly mixing the foam of the first agent and the foam of the second agent easily, and obtaining a foam which makes the operation of refoaming easier to be carried out, it is preferable that the respective mass ratio of the liquid concentrate : propellant in both the first agent and the second agent be 99:1 to 80:20, and more preferably 98:2 to 82:18, or 95: 5 to 85:15.

### EXAMPLES

### Reference Examples 1 to 9 and Examples 1 to 3

A first agent liquid concentrate and a second agent liquid concentrate as indicated in Table 1 and Table 2 were prepared, and the liquid concentrates were respectively filled, together with a propellant, in an aerosol container at a mass ratio of liquid concentrate: propellant of 90 : 10. LPG at 0.20 MPa (20°C) was used in Reference Examples 1 to 8 and Examples 1 to 3, and DME/LPG (40/60) (20°C) at 0.20 MPa was used in Reference Example 9, as propellant. The amounts of the liquid concentrate of the first agent and the second agent were both set to 50 g (total amount 100 g) .

**[Table 1]**

| | **R**ef. Example 1 | Ref. Example 2 | Ref. Example 3 | Ref. Example 4 | Example 1 | Ref. Example 5 | Ref. Example 6 | Ref. Example 7 | Example 2 |
|---|---|---|---|---|---|---|---|---|---|
| First agent liquid concentrate (mass%) | B | C | D | E | F | G | H | I | J |
| Toluene-2,5-diamine solution (20%) | 2.50 | 2.50 | 2.50 | 2.50 | 2.50 | 2.50 | 2.50 | 2.50 | 2.50 |
| Resorcin | 0.50 | 0.50 | 0.50 | 0.50 | 0.50 | 0.50 | 0.50 | 0.50 | 0.50 |
| Sodium cocoyl glutamate solution (24%) | 8.34 | 41.70 | 41.70 | - | - | - | - | - | - |
| Lauramidopropyl betaine (30%) | - | - | - | 6.67 | 33.35 | 33.35 | - | - | - |
| Sodium polyoxyethylene(4.5) lauryl ether acetate (24%) | - | - | - | - | - | - | 41.70 | - | - |
| Sodium polyoxyethylene(2) lauryl ether sulfate (27%) | - | - | - | - | - | - | - | 37.03 | - |
| Lauryl hydroxysulfobetaine (30%) | - | - | - | - | - | - | - | - | 33.35 |
| Polyoxyethylene(40) cetyl ether | 0.46 | 0.46 | - | 0.46 | 0.46 | - | 0.46 | 0.46 | 0.46 |
| Propylene qlvcol | 4.00 | 4.00 | 4.00 | 4.00 | 4.00 | 4.00 | 4.00 | 4.00 | 4.00 |
| Ammonium hydroxide (28%) | 3.00 | 3.00 | 3.00 | 3.00 | 3.00 | 3.00 | 3.00 | 3.00 | 3.00 |
| Ascorbic acid | 0.40 | 0.40 | 0.40 | 0.40 | 0.40 | 0.40 | 0.40 | 0.40 | 0.40 |
| Anhydrous sodium sulfite | 0.40 | 0.40 | 0.40 | 0.40 | 0.40 | 0.40 | 0.40 | 0.40 | 0.40 |
| Tetrasodium edetate dihydrate | 0.10 | 0.10 | 0.10 | 0.10 | 0.10 | 0.10 | 0.10 | 0.10 | 0.10 |
| Purified water | Balance | Balance | Balance | Balance | Balance | Balance | Balance | Balance | Balance |

| Second agent liquid concentrate (mass%) | A | A | B | A | A | B | A | A | A |
|---|---|---|---|---|---|---|---|---|---|
| Stearyltrimethylammonium chloride (28%) | 2.51 | 2.51 | - | 2.51 | 2.51 | - | 2.51 | 2.51 | 2.51 |
| Polyoxyethylene(40) cetyl ether | 0.46 | 0.46 | 0.92 | 0.46 | 0.46 | 0.92 | 0.46 | 0.46 | 0.46 |
| Cetanol | 0.74 | 0.74 | 0.74 | 0.74 | 0.74 | 0.74 | 0.74 | 0.74 | 0.74 |
| Myristyl alcohol | 0.21 | 0.21 | 0.21 | 0.21 | 0.21 | 0.21 | 0.21 | 0.21 | 0.21 |
| Hydroxyethanediphosphonic acid solution (60%) | 0.07 | 0.07 | 0.07 | 0.07 | 0.07 | 0.07 | 0.07 | 0.07 | 0.07 |
| Oxyquinoline(2) sulfate | 0.04 | 0.04 | 0.04 | 0.04 | 0.04 | 0.04 | 0.04 | 0.04 | 0.04 |
| 48% sodium hydroxide solution | 0.03 | 0.03 | 0.03 | 0.03 | 0.03 | 0.03 | 0.03 | 0.03 | 0.03 |
| 35% aqueous hydrogen peroxide | 16.29 | 16.29 | 16.29 | 16.29 | 16.29 | 16.29 | 16.29 | 16.29 | 16.29 |
| Purified water | Balance | Balance | Balance | Balance | Balance | Balance | Balance | Balance | Balance |

| Propellant (common to first agent, second agent) | LPG | LPG | LPG | LPG | LPG | LPG | LPG | LPG | LPG |
|---|---|---|---|---|---|---|---|---|---|
| Liquid concentrate : propellant (mass ratio) | 90:10 | 90:10 | 90:10 | 90:10 | 90:10 | 90:10 | 90:10 | 90:10 | 90:10 |

**[Table 2]**

| | Ref. Example 8 | Example 3 | Ref. Example 9 |
|---|---|---|---|
| First agent (mass%) | K | L | K |
| Stearyltrimethylammonium chloride (28%) | 1.26 | 1.26 | 1.26 |
| Polyoxyethylene(40) cetyl ether | 0.23 | 0.23 | 0.23 |
| Cetanol | 0.37 | 0.37 | 0.37 |
| Myristyl alcohol | 0.11 | 0.11 | 0.11 |
| Hydroxyethanediphosphonic acid solution (60%) | 0.04 | 0.04 | 0.04 |
| Oxyquinoline(2) sulfate | 0.020 | 0.020 | 0.020 |
| 48% sodium hydroxide solution | 0.015 | 0.015 | 0.015 |
| Sodium cocoyl glutamate solution (24%) | 20.85 | - | 20.85 |
| Lauramidopropyl betaine (30%) | - | 16.68 | - |
| Toluene-2,5-diamine solution (20%) | 2.50 | 2.50 | 2.50 |
| Resorcin | 0.50 | 0.50 | 0.50 |
| Propylene glycol | 4.00 | 4.00 | 4.00 |
| Ammonium hydroxide (28%) | 3.00 | 3.00 | 3.00 |
| Ascorbic acid | 0.40 | 0.40 | 0.40 |
| Anhydrous sodium sulfite | 0.40 | 0.40 | 0.40 |
| Tetrasodium edetate dihydrate | 0.10 | 0.10 | 0.10 |
| Purified water | Balance | Balance | Balance |

| Second agent (mass%) | C | D | C |
|---|---|---|---|
| Stearyltrimethylammonium chloride (28%) | 1.26 | 2.51 | 1.26 |
| Polyoxyethylene(40) cetyl ether | 0.23 | 0.46 | 0.23 |
| Cetanol | 0.37 | 0.74 | 0.37 |
| Myristyl alcohol | 0.11 | 0.21 | 0.11 |
| Hydroxyethanediphosphonic acid solution (60%) | 0.04 | 0.07 | 0.04 |
| Oxyquinoline(2) sulfate | 0.020 | 0.04 | 0.020 |
| 48% sodium hydroxide liquid | 0.015 | 0.03 | 0.015 |
| Sodium cocoyl glutamate solution (24%) | 20.85 | - | 20.85 |
| Lauramidopropyl betaine (30%) | - | 16.68 | - |
| 35% aqueous hydrogen peroxide | 16.29 | 16.29 | 16.29 |
| Purified water | Balance | Balance | Balance |

| Propellant (common to first agent and second agent) | LPG | LPG | DME/LPG (40/60) |
|---|---|---|---|
| Liquid concentrate : propellant (mass ratio) | 90:10 | 90:10 | 90:10 |

The following were used for evaluation.
- Hair: A wig with uniform semi-long hair (Beaulax Co., Ltd., product No. 775S)
- Aerosol container: 100-mL aerosol test bottle, manufactured by Tokyo Kobunshi Co., Ltd., was used for both first agent and second agent

### [Hair dyeing method used in evaluation]

Hair dyeing treatment for the wig was carried out by an ordinary person who was not skillful with the procedure of hair dyeing.

Aerosol containers are respectively filled with one of the liquid concentrates and the propellant, and then the containers are sufficiently shaken. After confirmation of the liquid concentrate and the propellant becoming uniform, the contents are discharged. The foam of the first agent and the foam of the second agent were simultaneously held in one hand wearing a glove, and immediately thereafter, the agents were applied to the hair of the wig used for the evaluation. The step of discharging the contents of the aerosol containers, holding the contents in one hand, and applying the contents to the head hair, was repeated, and thus all of the hair dye in the aerosol containers was applied to the hair.

Immediately after completion of the application, the entire hair is rubbed for 20 seconds using the fingers of both hands wearing gloves, and thus the applied foam of the first agent and the foam of the second agent are refoamed.

Subsequently, the hair dye is left to stand still for 10 minutes.

Subsequently, the step of refoaming is carried out for 30 seconds.

Subsequently, the hair dye is left to stand still until a lapse of 30 minutes counting from the completion of the application of the hair dye.

The entire head hair is washed with warm water, and the applied hair dye is washed away. The hair is washed two times using shampoo, and conditioner is applied once. The hair is washed away and dried.

### [Evaluation]

The evaluation on the "ease of refoaming" was carried out by persons who carried out the hair dyeing operation (4 persons), and the evaluations on the "hair dyeability" and "color uniformity" were carried out through visual inspection by skilled panelists (4 persons) . The following scores of 5 grades were defined as the evaluation criteria, and the evaluation was scored by 0.5 points in unit. That is, when each evaluator was undetermined about which grade should be assigned to the results of evaluation, for example, when the evaluator can not determine which grade, 5 or 4, should be assigned, the evaluator was allowed to assign grade 4.5.

The results are shown in Table 3, as average values of the results of 4 persons.

### "Ease of refoaming"

- 5:: Foaming was very well
- 4:: Foaming was slightly well
- 3:: Foaming was neither well nor poor
- 2:: Foaming was slightly poor
- 1:: Foaming was not observed at all

### "Dyeability"

- 5:: Very well
- 4:: Slightly well
- 3:: Neither well nor poor
- 2:: Slightly poor
- 1:: Poor

### "Color uniformity"

- 5:: Very well
- 4:: Slightly well
- 3:: Neither well nor poor
- 2:: Slightly poor
- 1:: Poor

**[Table 3]**

| | Reference Examples 1-9 and Examples 1-3 | | | | | | | | | | | |
|---|---|---|---|---|---|---|---|---|---|---|---|---|
| | Ref. Ex. 1 | Ref. Ex. 2 | Ref. Ex. 3 | Ref. Ex. 4 | Ref. Ex. 5 | Ref. Ex. 6 | Ref. Ex. 7 | Ref. Ex. 8 | Ref. Ex. 9 | Ex 1 | Ex 2 | Ex 3 |
| Ease of refoaming | 3.50 | 4.25 | 4.50 | 3.75 | 4.50 | 4.50 | 4.50 | 5.00 | 5.00 | 5.00 | 5.00 | 5.00 |
| Dyeability | 5.00 | 4.00 | 4.00 | 5.00 | 4.38 | 4.38 | 4.38 | 4.38 | 4.38 | 5.00 | 5.00 | 5.00 |
| Color uniformity | 3.25 | 5.00 | 4.25 | 3.25 | 4.50 | 4.25 | 4.50 | 4.75 | 5.00 | 5.00 | 5.00 | 5.00 |

### Comparative Example 1

Hair dyeing was carried out using the same first agent liquid concentrate L, the same second agent liquid concentrate D, and the same propellant (LPG) in the same amounts and proportions, and in the same manner as in Example 3, except that foams were applied to the hair during the hair dyeing procedure, and then the hair dye was left to stand still for 30 minutes without refoaming.

Thereafter, the evaluations were carried out in the same manner, and the following results were obtained.

**[Table 4]**

| | Comparative Example 1 |
|---|---|
| Ease of refoaming | - |
| Dyeability | 4.50 |
| Color uniformity | 1.25 |

## Claims

1. A method of dyeing hair comprising the steps of discharging a first agent and a second agent from the respective containers of an aerosol foam hair dye composed of the following first agent and second agent, applying the discharged agents to hair, and then refoaming the agents on the hair:
a first agent contained in a first aerosol container, including a first agent liquid concentrate containing components (A), (C) and (D), and a propellant:
(A) an alkali agent,
(C) a polyoxyethylene alkyl ether type or polyoxyethylene alkenyl ether type nonionic surfactant, having an HLB value of 11 or more, and
(D) water; and
a second agent contained in a second aerosol container, including a second agent liquid concentrate containing components (B), (C) and (D), and a propellant:
(B) hydrogen peroxide,
(C) a polyoxyethylene alkyl ether type or polyoxyethylene alkenyl ether type nonionic surfactant, having an HLB value of 11 or more, and
(D) water;
wherein the first agent and/or the second agent further contains an amphoteric surfactant; and wherein the content of surfactant in the liquid concentrates of the first agent and the second agent is 4 to 30% by mass.

2. The method of dyeing hair according to claim 1, wherein the aerosol foam hair dye contains an oil agent in an amount of 0.01% to 10% by mass based on the total amount of the first agent liquid concentrate and the second agent liquid concentrate.

3. The method of dyeing hair according to claim 1, wherein the aerosol foam hair dye contains an oil agent in an amount of 0.3% to 1.5% by mass based on the total amount of the first agent liquid concentrate and the second agent liquid concentrate.

4. The method of dyeing hair according to claim 2 or 3, wherein the mass ratio of the surfactant and the oil agent (surfactant/oil agent) in the whole mixture prepared by adding the first agent liquid concentrate and the second agent liquid concentrate of the aerosol foam hair dye is 2 to 3000.

5. The method of dyeing hair according to claim 2 or 3, wherein the mass ratio of the surfactant and the oil agent (surfactant/oil agent) in the whole mixture prepared by adding the first agent liquid concentrate and the second agent liquid concentrate of the aerosol foam hair dye is 5 to 100.

6. The method of dyeing hair according to any one of claims 1 to 5, wherein the viscosity of the mixed liquid of the first agent liquid concentrate and the second agent liquid concentrate of the aerosol foam hair dye is 10 to 10000 mPa·s.

7. The method of dyeing hair according to any one of claims 1 to 6, wherein the content of water in the total amount of the first agent liquid concentrate and the second agent liquid concentrate is 70% to 96% by mass.

8. The method of dyeing hair according to any one of claims 1 to 7, wherein the aerosol foam hair dye contains a solvent in an amount of 0.1% to 15% by mass based on the total amount of the first agent liquid concentrate and the second agent liquid concentrate.

9. The method of dyeing hair according to any one of claims 1 to 7, wherein the aerosol foam hair dye contains a solvent in an amount of 0.2% to 10% by mass based on the total amount of the first agent liquid concentrate and the second agent liquid concentrate.

10. The method of dyeing hair according to any one of claims 1 to 9, wherein the aerosol foam hair dye contains dimethyl ether as a propellant.

11. The method of dyeing hair according to any one of claims 1 to 10, wherein the content of surfactant in the liquid concentrates of the first agent and the second agent is 5% to 15% by mass.

12. The method of dyeing hair according to any one of claims 1 to 11, wherein the amphoteric surfactant is at least one member selected from the group consisting of carbobetaines, aminobetaines, sulfobetaines, hydroxysulfobetaines, amidosulfobetaines, phosphobetaines and imidazoliniums having an alkyl group, alkenyl group or acyl group having 8 to 24 carbon atoms.

## Patentansprüche

1. Verfahren zum Färben von Haar, das die Schritte des Entnehmens eines ersten Mittels und eines zweiten Mittels aus den jeweiligen Behältern für ein Aerosolschaum-Haarfärbemittel, das aus dem folgenden ersten Mittel und zweiten Mittel zusammengesetzt ist, des Anwendens der entnommenen Mittel auf Haar, und anschließend des Wiederaufschäumens der Mittel auf dem Haar umfasst:
ein erstes Mittel, das in einem ersten Aerosolbehälter enthalten ist und ein flüssiges Konzentrat des ersten Mittels enthält, das Komponenten (A), (C) und (D), und ein Treibmittel enthält:
(A) ein alkalisches Mittel
(C) ein nicht-ionisches Polyoxyethylenalkylether-Tensid oder Polyoxyethylenealkenylether-Tensid, das einen HLB-Wert von 11 oder mehr aufweist, und
(D) Wasser; und
ein zweites Mittel, das in einem zweiten Aerosolbehälter enthalten ist und ein flüssiges Konzentrat des zweiten Mittels enthält, das Komponenten (B), (C) und (D), und ein Treibmittel enthält:
(B) Wasserstoffperoxid,
(C) ein nicht-ionisches Polyoxyethylenalkylether-Tensid oder Polyoxyethylenealkenylether-Tensid, das einen HLB-Wert von 11 oder mehr aufweist, und
(D) Wasser;
wobei das erste Mittel und/oder das zweite Mittel ferner ein amphoteres Tensid enthält; und wobei der Tensidgehalt in den flüssigen Konzentraten des ersten Mittels und des zweiten Mittels 4 bis 30 Massen% beträgt.

2. Verfahren zum Färben von Haar gemäß Anspruch 1, wobei das Aerosolschaum-Haarfärbemittel ein öliges Mittel in einer Menge von 0,01 Massen% bis 10 Massen% in Bezug auf die Gesamtmenge des flüssigen Konzentrats des ersten Mittels und des flüssigen Konzentrats des zweiten Mittels enthält.

3. Verfahren zum Färben von Haar gemäß Anspruch 1, wobei das Aerosolschaum-Haarfärbemittel ein öliges Mittel in einer Menge von 0,3 Massen% bis 1,5 Massen% in Bezug auf die Gesamtmenge des flüssigen Konzentrats des ersten Mittels und des flüssigen Konzentrats des zweiten Mittels enthält.

4. Verfahren zum Färben von Haar gemäß Anspruch 2 oder 3, wobei das Massenverhältnis des Tensids und des öliges Mittels (Tensid/öliges Mittel) in der gesamten Mischung, die durch Hinzufügen des flüssigen Konzentrats des ersten Mittels und des flüssigen Konzentrats des zweiten Mittels des Aerosolschaum-Haarfärbemittels hergestellt wird, 2 bis 3000 beträgt.

5. Verfahren zum Färben von Haar gemäß Anspruch 2 oder 3, wobei das Massenverhältnis des Tensids und des öliges Mittels (Tensid/öliges Mittel) in der gesamten Mischung, die durch Hinzufügen des flüssigen Konzentrats des ersten Mittels und des flüssigen Konzentrats des zweiten Mittels des Aerosolschaum-Haarfärbemittels hergestellt wird, 5 bis 100 beträgt.

6. Verfahren zum Färben von Haar gemäß mindestens einem der Ansprüche 1 bis 5, wobei die Viskosität der gemischten Flüssigkeit des flüssigen Konzentrats des ersten Mittels und des flüssigen Konzentrats des zweiten Mittels des Aerosolschaum-Haarfärbemittels 10 bis 10000 mPa·s beträgt.

7. Verfahren zum Färben von Haar gemäß mindestens einem der Ansprüche 1 bis 6, wobei der Wassergehalt in der Gesamtmenge des flüssigen Konzentrats des ersten Mittels und des flüssigen Konzentrats des zweiten Mittels 70 Massen% bis 96 Massen% beträgt.

8. Verfahren zum Färben von Haar gemäß mindestens einem der Ansprüche 1 bis 7, wobei das Aerosolschaum-Haarfärbemittel ein Lösungsmittel in einer Menge von 0,1 Massen% bis 15 Massen% in Bezug auf die Gesamtmenge des flüssigen Konzentrats des ersten Mittels und des flüssigen Konzentrats des zweiten Mittels enthält.

9. Verfahren zum Färben von Haar gemäß mindestens einem der Ansprüche 1 bis 7, wobei das Aerosolschaum-Haarfärbemittel ein Lösungsmittel in einer Menge von 0,2 Massen% bis 10 Massen% in Bezug auf die Gesamtmenge des flüssigen Konzentrats des ersten Mittels und des flüssigen Konzentrats des zweiten Mittels enthält.

10. Verfahren zum Färben von Haar gemäß mindestens einem der Ansprüche 1 bis 9, wobei das Aerosolschaum-Haarfärbemittel Dimethylether als Treibmittel enthält.

11. Verfahren zum Färben von Haar gemäß mindestens einem der Ansprüche 1 bis 10, wobei der Tensidgehalt in den flüssigen Konzentraten des ersten Mittels und des zweiten Mittels 5 Massen% bis 15 Massen% beträgt.

12. Verfahren zum Färben von Haar gemäß mindestens einem der Ansprüche 1 bis 11, wobei das amphotere Tensid mindestens ein Vertreter, ausgewählt aus der Gruppe, bestehend aus Carbobetainen, Aminobetainen, Sulfobetainen, Hydroxysulfobetainen, Amidosulfobetainen, Phosphobetainen und aus Imidazolinen mit einer Alkylgruppe, Alkenylgruppe oder Acylgruppe mit 8 bis 24 Kohlenstoffatomen, ist.

## Revendications

1. Procédé de coloration capillaire comprenant les étapes consistant à décharger un premier agent et un second agent à partir des contenants respectifs d'un colorant capillaire en mousse aérosol composé du premier agent et du second agent suivants, à appliquer les agents déchargés sur les cheveux, et ensuite à remousser les agents sur les cheveux :
un premier agent contenu dans un premier contenant pressurisé, incluant un concentré liquide du premier agent contenant des composants (A), (C) et (D), et un propulseur :
(A) un agent alcalin,
(C) un tensioactif non ionique de type polyoxyéthylène alkyle éther ou polyoxyéthylène alcényle éther, ayant une valeur HLB de 11 ou plus, et
(D) de l'eau ; et
un second agent contenu dans un second contenant pressurisé, incluant un concentré liquide du second agent contenant les composants (B), (C) et (D), et un propulseur :
(B) du peroxyde d'hydrogène,
(C) un tensioactif non ionique de type polyoxyéthylène alkyle éther ou polyoxyéthylène alcényle éther, ayant une valeur HLB de 11 ou plus, et
(D) de l'eau,
dans lequel le premier agent et/ou le second agent contiennent en outre un tensioactif amphotère ; et dans lequel la teneur en tensioactif dans les concentrés liquides du premier agent et du second agent est de 4 à 30 % en masse.

2. Procédé de coloration capillaire selon la revendication 1, dans lequel le colorant capillaire en mousse aérosol contient un agent huileux en une quantité de 0,01 % à 10 % en masse sur la base de la quantité totale du concentré liquide du premier agent et du concentré liquide du second agent.

3. Procédé de coloration capillaire selon la revendication 1, dans lequel le colorant capillaire en mousse aérosol contient un agent huileux en une quantité de 0,3 % à 1,5 % en masse sur la base de la quantité totale du concentré liquide du premier agent et du concentré liquide du second agent.

4. Procédé de coloration capillaire selon l'une quelconque des revendications 2 ou 3, dans lequel le rapport massique du tensioactif et de l'agent huileux (agent tensioactif/ agent huileux) dans la totalité du mélange préparé en ajoutant le concentré liquide du premier agent et le concentré liquide du second agent du colorant capillaire en mousse aérosol est de 2 à 3 000.

5. Procédé de coloration capillaire selon l'une quelconque des revendications 2 ou 3, dans lequel le rapport massique du tensioactif et de l'agent huileux (agent tensioactif/agent huileux) dans la totalité du mélange préparé en ajoutant le concentré liquide du premier agent et le concentré liquide du second agent du colorant capillaire en mousse aérosol est de 5 à 100.

6. Procédé de coloration capillaire selon l'une quelconque des revendications 1 à 5, dans lequel la viscosité du liquide mélangé du concentré liquide du premier agent et du concentré liquide du second agent du colorant capillaire en mousse aérosol est de 10 à 10 000 mPa·s.

7. Procédé de coloration capillaire selon l'une quelconque des revendications 1 à 6, dans lequel la teneur en eau dans la quantité totale du concentré liquide du premier agent et du concentré liquide du second agent est de 70 % à 96 % en masse.

8. Procédé de coloration capillaire selon l'une quelconque des revendications 1 à 7, dans lequel le colorant capillaire en mousse aérosol contient un solvant en une quantité de 0,1 % à 15 % en masse sur la base de la quantité totale du concentré liquide du premier agent et du concentré liquide du second agent.

9. Procédé de coloration capillaire selon l'une quelconque des revendications 1 à 7, dans lequel le colorant capillaire en mousse aérosol contient un solvant en une quantité de 0,2 % à 10 % en masse sur la base de la quantité totale du concentré liquide du premier agent et du concentré liquide du second agent.

10. Procédé de coloration capillaire selon l'une quelconque des revendications 1 à 9, dans lequel le colorant capillaire en mousse aérosol contient de l'éther diméthylique comme agent propulseur.

11. Procédé de coloration capillaire selon l'une quelconque des revendications 1 à 10, dans lequel la teneur en tensioactif dans les concentrés liquides du premier agent et du second agent est de 5 % à 15% en masse.

12. Procédé de coloration capillaire selon l'une quelconque des revendications 1 à 11, dans lequel le tensioactif amphotère est au moins un élément choisi dans le groupe qui consiste en carbobétaïnes, amidobétaïnes, sulfobétaïnes, hydroxysulfobétaïnes, amidosulfobétaïnes, phosphobétaïnes et imidazoliniums ayant un groupe alkyle, un groupe alcényle ou un groupe acyle ayant de 8 à 24 atomes de carbone.
